# EUROPEAN PATENT SPECIFICATION

(11) **EP 3 568 081 B1**
(45) Date of publication and mention of the grant of the patent: **29.11.2023**
(21) Application number: 18706637.8
(22) Date of filing: 04.01.2018
(51) Int. Cl.: A61B 8/12, A61B 8/00

(54) **SUPPORT MEMBERS FOR CONNECTION OF COMPONENTS IN INTRALUMINAL DEVICES, SYSTEMS, AND METHODS**
TRÄGERELEMENTE ZUR VERBINDUNG VON KOMPONENTEN IN INTRALUMINALEN VORRICHTUNGEN, SYSTEME UND VERFAHREN
ÉLÉMENTS DE SUPPORT DE CONNEXION DE COMPOSANTS DANS DES DISPOSITIFS INTRALUMINAUX, SYSTÈMES ET PROCÉDÉS

(30) Priority: 12.01.2017 US 201762445453 P
(43) Date of publication of application: 20.11.2019
(73) Proprietor: Koninklijke Philips N.V., 5656 AG Eindhoven (NL)
(72) Inventor: MINAS, Maritess, 5656 AE Eindhoven (NL); STIGALL, Jeremy, 5656 AE Eindhoven (NL); SAROHA, Princeton, 5656 AE Eindhoven (NL); WROLSTAD, David, Kenneth, 5656 AE Eindhoven (NL)
(74) Representative: Philips Intellectual Property & Standards
(86) International application number: PCT/EP2018/050158
(87) International publication number: WO 2018/130449

(56) References cited:
- US-A- 4 917 097
- US-A- 5 947 905
- US-A1- 2015 305 710

## Description

### TECHNICAL FIELD

The present disclosure relates generally to intraluminal imaging and, in particular, to connecting components of an intraluminal imaging device. For example, an intraluminal imaging device can include a support member positioned over a junction between an imaging component and a catheter shaft. The connecting support member can be structured to provide durability, strength, and a complete electrical seal at the junction while maintaining a low catheter profile.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state (also known as synthetic-aperture) IVUS catheters are one of the two types of IVUS devices commonly used today, the other type being the rotational IVUS catheter. Solid-state IVUS catheters carry a scanner assembly that includes an array of ultrasound transducers distributed around its circumference along with one or more integrated circuit controller chips mounted adjacent to the transducer array. The controllers select individual transducer elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

The electrical cable and the solid-state scanner are connected during assembly of the IVUS device. Generally, this requires that conductors in the electrical cable be aligned with a respective conductive pad on the solid-state scanner and soldered together. Often the soldering of the conductors to the conductive pads is the only mechanical connection between the electrical cable and the solid-state scanner. Further, an epoxy or other adhesive is used to encase the soldered electrical connections and also to couple the imaging assembly to a shaft of the catheter. In this regard, often the resulting glob of epoxy is shaped or smoothed in an effort to define a relatively smooth transition across the joint between the shaft and the imaging assembly. However, this process is subject to high variation in the resulting outer profile of the device and connection strength from one assembly person to the next and even between devices assembled by the same person. The inconsistent profile and connection strength at the junction between the imaging assembly and the catheter shaft may impact the navigational capability and the imaging reliability of intraluminal imaging devices.
A document US 4 917 097 A provides an in vivo imaging device for producing real-time images of small, moving or stationary cavities and surrounding issue structure. The imaging device includes a probe assembly of very small dimensions and preferably sufficiently small to fit within cavities having a diameter on the order of that of a human coronary artery. The probe assembly may be mounted to a positioning device such as a catheter, which allows for the use of, for example, conventional guiding catheters and guide wires.
Another document US 2015/0305710 A1 relates to systems, devices, and methods to provide a solid-state intravascular ultrasound (IVUS) imaging system that includes an array of ultrasound transducers mounted on a structural uni-body made of a polymeric substance doped with acoustic dampening material. The use of the polymeric substance doped with acoustic dampening material to fabricate the uni-body assists in improving the signal-to-noise ratio associated with the IVUS imaging signals.
Another document US 5 947 905 A relates to an ultrasound transducer array probe for intraluminal ultrasound imaging, which is situated on a distal end of a catheter. The probe has a flex circuit that accepts terminations from a plurality of coaxial cables admitted through a catheter, and electrically conveys these signals to integrated circuits and an ultrasound array acoustic stack, preferably having seventy-two elements. The circuit has more than one layer of metal tracings to support complicated electrical interconnections. The acoustic stack preferably includes two quarter-wave matching layers and an acoustic backing layer composed of urethane, AIN3, tungsten trioxide, and micro-balloons. The flex circuit can be formed of two sections that are joined during manufacture.

### SUMMARY

The object of the present invention is solved by the subject-matter of the independent claims; further embodiments are incorporated in the dependent claims. Embodiments of the present disclosure provide a connecting support member for interconnecting an imaging assembly and a catheter shaft. The connecting support member is placed over a distal portion of an outer catheter shaft and bonded over a proximal portion of the imaging assembly. The connecting support member is at least partially overlapped with the outer catheter shaft and the imaging assembly to create a consistent smooth transition between the outer catheter shaft and the imaging assembly. The connecting support member can increase the mechanical strength at the interconnection while maintaining a low profile. The connecting support member can be molded such that one end matches the diameter and shape of the imaging assembly and another end matches the diameter and shape of the outer catheter shaft. Thus, the connecting support member can provide a complete electrical seal at the interconnection and prevent liquid ingress that may degrade imaging quality or cause imaging failure.

According to a first aspect, an intraluminal imaging device is provided. The intraluminal imaging device comprises a flexible elongate member configured for positioning within a vessel of a patient; a support member coupled to the flexible elongate member; and an imaging assembly coupled to the support member. The support member includes a proximal section with a first cross-sectional profile configured to interface with a distal portion of the flexible elongate member and a distal section with a second cross-sectional profile configured to interface with a proximal portion of the imaging assembly, wherein the first cross-sectional profile is different than the second cross-sectional profile.

The first cross-sectional profile is circular. The second cross-sectional profile is a geometrical shape including between four and twelve sides. In some embodiments, the support member further includes a lumen and an opening extending through a wall in communication with the lumen, the opening configured to receive a communication cable. In some embodiments, a connection interface of the imaging assembly is mounted to an outer surface of the support member adjacent to the opening. In some embodiments, the connection interface includes a flex circuit. In some embodiments, the intraluminal imaging device includes the communication cable extending through the opening and electrically coupled to the connection interface of the imaging assembly. In some embodiments, the support member includes a lumen and an adhesive within the lumen fixedly secures the imaging assembly to the support member. In some embodiments, a connection interface of the imaging assembly is positioned within the lumen. In some embodiments, the imaging assembly comprises an ultrasound transducer array.

According to a first aspect, a method of forming an intraluminal imaging device is provided. The method includes providing a support member comprising a proximal section with a first cross-sectional profile and a distal section with a second cross-sectional profile, wherein the first cross-sectional profile is different than the second cross-sectional profile; coupling a distal portion of a flexible elongate member configured for positioning within a vessel of a patient to the proximal section of the support member; and coupling a proximal portion of an imaging assembly to the distal section of the support member.

In some embodiments, the support member includes a lumen and an opening extending through a wall in communication with the lumen, and the method further comprises introducing a communication cable through the opening. In some embodiments, the method further comprises mounting a connection interface of the imaging assembly to an outer surface of the support member adjacent to the opening. In some embodiments, the method further comprises electrically coupling the communication cable to the connection interface of the imaging assembly. In some embodiments, the method further comprises introducing an adhesive within a lumen of the support member to fixedly secure the imaging assembly to the support member. In some embodiments, the method further comprises positioning a connection interface of the imaging assembly within the lumen. In some embodiments, the connection interface is electrically coupled to a communication cable extending at least partially through the lumen.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a diagrammatic schematic view of an imaging system, according to aspects of the present disclosure.
Fig. 2 is a diagrammatic top view of a scanner assembly in a flat configuration, according to aspects of the present disclosure.
Fig. 3 is a diagrammatic side view of a scanner assembly in a rolled configuration around a support member, according to aspects of the present disclosure.
Fig. 4 is a diagrammatic side view of a support member, according to aspects of the present disclosure.
Fig. 5 is a diagrammatic cross-sectional side view of the support member of Fig. 4, according to aspects of the present disclosure.
Fig. 6 is a diagrammatic end view of the support member of Figs. 4 and 5, according to aspects of the present disclosure.
Fig. 7 is a diagrammatic side view of a distal portion of an intraluminal imaging device in an assembly stage, according to aspects of the present disclosure.
Fig. 8 is a diagrammatic partial cross-sectional side view of the distal portion of the intraluminal imaging device in the assembly stage of Fig. 7, according to aspects of the present disclosure.
Fig. 9 is a diagrammatic side view of a distal portion of an intraluminal imaging device, according to aspects of the present disclosure.
Fig. 10 is a diagrammatic side view of a support member, according to aspects of the present disclosure.
Fig. 11 is a diagrammatic cross-sectional side view of the support member of Fig. 10, according to aspects of the present disclosure.
Fig. 12 is a diagrammatic end view of the support member of Figs. 10 and 11, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

The intraluminal imaging devices described herein achieve numerous advantages. For example, the support members of the intraluminal imaging devices facilitate a faster, less labor-intensive, and more efficient manufacturing process for the intraluminal device. Additionally, coupling a flexible elongate member to an imaging assembly using the support member creates a more robust imaging assembly less prone to breakage. For example, each end of the support member can be shaped to mate with corresponding surfaces of the flexible elongate member and the imaging assembly. In this regard, the support member can also define a support surface to which a portion of a flex circuit of the imaging assembly containing conductive pads or traces can be mounted for electrical connection to a communication cable. As a result, an improved solder/weld interface between the wires and the conductive pads can be created while also improving the mechanical coupling between the flexible elongate member and the imaging assembly.

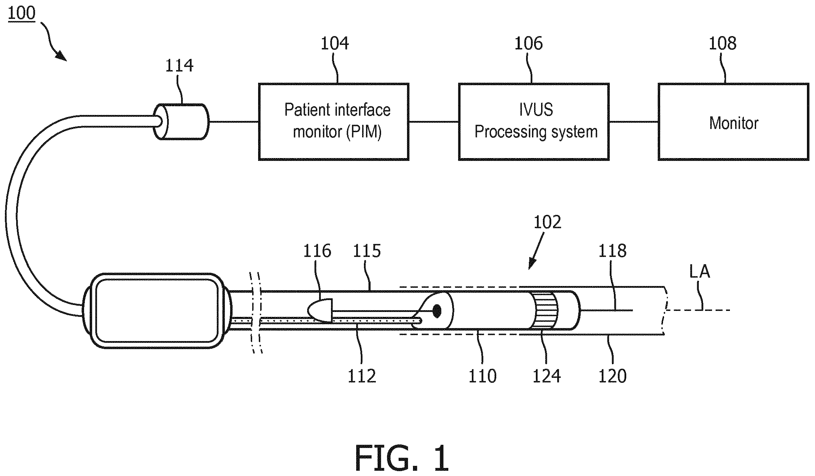

Fig. 1 is a diagrammatic schematic view of an intravascular ultrasound (IVUS) imaging system 100, according to aspects of the present disclosure. The IVUS imaging system 100 may include a solid-state IVUS device 102 such as a catheter, guide wire, or guide catheter, a patient interface module (PIM) 104, an IVUS processing system or console 106, and a monitor 108.

At a high level, the IVUS device 102 emits ultrasonic energy from a transducer array 124 included in imaging assembly 110 mounted near a distal end of the catheter device. The ultrasonic energy is reflected by tissue structures in the medium, such as a vessel 120, surrounding the imaging assembly 110, and the ultrasound echo signals are received by the transducer array 124. The PIM 104 transfers the received echo signals to the processing system 106 where the ultrasound image (including the flow information) is reconstructed and displayed on the monitor 108. The processing system 106 can include a processor and a memory. The processing system 106 can be operable to facilitate the features of the IVUS imaging system 100 described herein. For example, the processor can execute computer readable instructions stored on the non-transitory tangible computer readable medium.

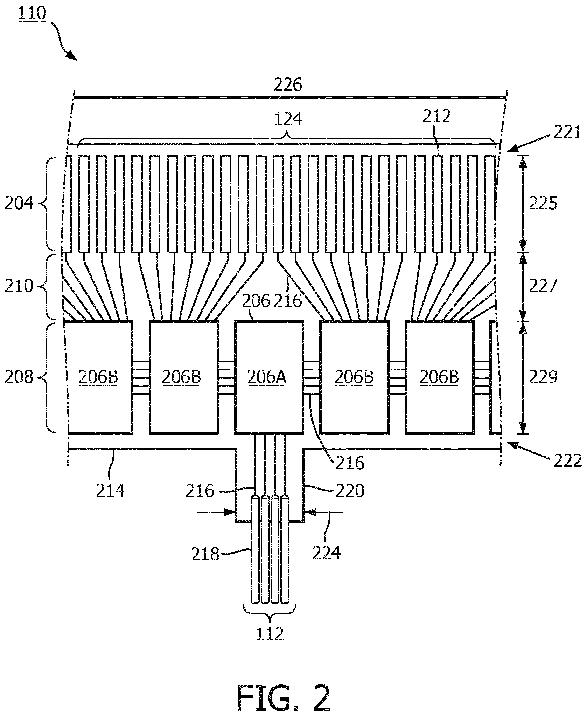

The PIM 104 facilitates communication of signals between the processing system 106 and the imaging assembly 110 included in the IVUS device 102. This communication includes the steps of: (1) providing commands to integrated circuit controller chip(s) 206A, 206B, illustrated in Fig. 2, included in the imaging assembly 110 to select the particular transducer array element(s) to be used for transmit and receive, (2) providing the transmit trigger signals to the integrated circuit controller chip(s) 206A, 206B included in the imaging assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s) of the imaging assembly 110. In some embodiments, the PIM 104 performs preliminary processing of the echo data prior to relaying the data to the processing system 106. In examples of such embodiments, the PIM 104 performs amplification, filtering, and/or aggregating of the data. In an embodiment, the PIM 104 also supplies high- and low-voltage DC power to support operation of the device 102 including circuitry within the imaging assembly 110.

The processing system 106 receives the echo data from the imaging assembly 110 by way of the PIM 104 and processes the data to reconstruct an image of the tissue structures in the medium surrounding the imaging assembly 110. The processing system 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Volcano Corporation and those disclosed in U.S. Patent No. 7,846,101. For example, the IVUS device 102 includes the imaging assembly 110 near a distal end of the device 102 and a cable 112 extending along the longitudinal body of the device 102. The cable 112 can include a plurality of conductors, including one, two, three, four, five, six, seven, or more conductors 218 (Fig. 2). It is understood that any suitable gauge wire can be used for the conductors 218. In an embodiment, the cable 112 can include a four-conductor transmission line arrangement with, e.g., 41 AWG gauge wires (0.07 mm diameter). In an embodiment, the cable 112 can include a seven-conductor transmission line arrangement utilizing, e.g., 44 AWG gauge wires (0.05 mm diameter). In some embodiments, 43 AWG gauge wires (0.056 mm diameter) can be used.

The cable 112 terminates in a PIM connector 114 at a proximal end of the device 102. The PIM connector 114 electrically couples the cable 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

The IVUS device 102 includes a flexible elongate member 115 having a proximal portion and a distal portion. The imaging assembly 110 is positioned at a distal portion of the flexible elongate member 115. The flexible elongate member 115 includes a longitudinal axis LA. The longitudinal axis LA may be associated with the IVUS device 102 and/or the imaging assembly 110.

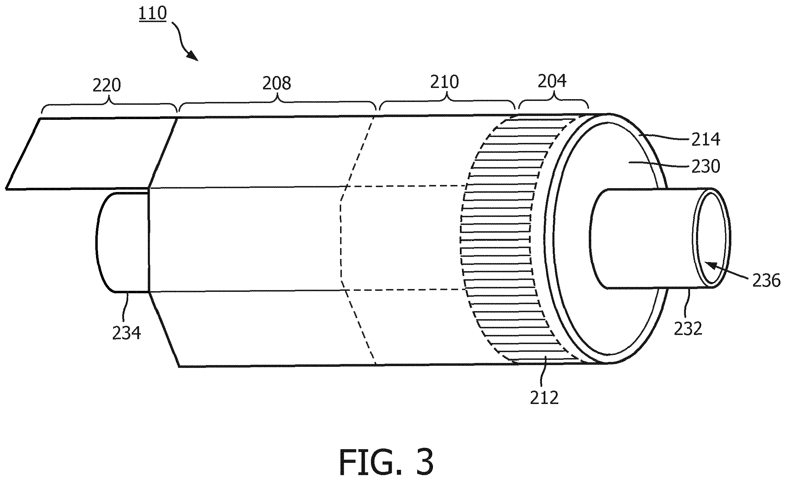

Fig. 2 is a top view of a portion of an ultrasound imaging assembly 110 according to an embodiment of the present disclosure. The imaging assembly 110 includes a transducer array 124 formed in a transducer region 204 and transducer control logic dies 206 (including dies 206A and 206B) formed in a control region 208, with a transition region 210 disposed therebetween. The transducer control logic dies 206 and the transducers 212 are mounted on a flex circuit 214 that is shown in a flat configuration in Fig. 2. Fig. 3 illustrates a rolled configuration of the flex circuit 214. The transducer array is a non-limiting example of a medical sensor element and/or a medical sensor element array. The transducer control logic dies 206 is a non-limiting example of a control circuit. The transducer region 204 is disposed adjacent a distal portion 221 of the flex circuit 214. The control region 208 is disposed adjacent the proximal portion 222 of the flex circuit 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or a length 227 of the transition region 210 can be greater than lengths 225, 229 of the transducer region and controller region, respectively. While the imaging assembly 110 is described as including a flex circuit, it is understood that the transducers and/or controllers may be arranged to form the imaging assembly 110 in other configurations, including those omitting a flex circuit.

The transducer array 124 may include any number and type of ultrasound transducers 212, although for clarity only a limited number of ultrasound transducers are illustrated in Fig. 2. In an embodiment, the transducer array 124 includes 64 individual ultrasound transducers 212. In a further embodiment, the transducer array 124 includes 32 ultrasound transducers 212. Other numbers are both contemplated and provided for. With respect to the types of transducers, in an embodiment, the ultrasound transducers 212 are piezoelectric micromachined ultrasound transducers (PMUTs) fabricated on a microelectromechanical system (MEMS) substrate using a polymer piezoelectric material, for example as disclosed in U.S. Patent 6,641,540. In alternate embodiments, the transducer array includes piezoelectric zirconate transducers (PZT) transducers such as bulk PZT transducers, capacitive micromachined ultrasound transducers (cMUTs), single crystal piezoelectric materials, other suitable ultrasound transmitters and receivers, and/or combinations thereof.

The imaging assembly 110 may include various transducer control logic, which in the illustrated embodiment is divided into discrete control logic dies 206. In various examples, the control logic of the imaging assembly 110 performs: decoding control signals sent by the PIM 104 across the cable 112, driving one or more transducers 212 to emit an ultrasonic signal, selecting one or more transducers 212 to receive a reflected echo of the ultrasonic signal, amplifying a signal representing the received echo, and/or transmitting the signal to the PIM across the cable 112. In the illustrated embodiment, a imaging assembly 110 having 64 ultrasound transducers 212 divides the control logic across nine control logic dies 206, of which five are shown in Fig. 2. Designs incorporating other numbers of control logic dies 206 including 8, 9, 16, 17 and more are utilized in other embodiments. In general, the control logic dies 206 are characterized by the number of transducers they are capable of driving, and exemplary control logic dies 206 drive 4, 8, and/or 16 transducers.

The control logic dies are not necessarily homogenous. In some embodiments, a single controller is designated a master control logic die 206A and contains the communication interface for the cable 112. Accordingly, the master control circuit may include control logic that decodes control signals received over the cable 112, transmits control responses over the cable 112, amplifies echo signals, and/or transmits the echo signals over the cable 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

The flex circuit 214, on which the transducer control logic dies 206 and the transducers 212 are mounted, provides structural support and interconnects for electrical coupling. The flex circuit 214 may be constructed to include a film layer of a flexible polyimide material such as KAPTON^{™} (trademark of DuPont). Other suitable materials include polyester films, polyimide films, polyethylene napthalate films, or polyetherimide films, other flexible printed semiconductor substrates as well as products such as Upilex^{®} (registered trademark of Ube Industries) and TEFLON^{®} (registered trademark of E.I. du Pont). In the flat configuration illustrated in Fig. 2, the flex circuit 214 has a generally rectangular shape. As shown and described herein, the flex circuit 214 is configured to be wrapped around a support member 230 (Fig. 3) to form a cylindrical toroid in some instances. Therefore, the thickness of the film layer of the flex circuit 214 is generally related to the degree of curvature in the final assembled imaging assembly 110. In some embodiments, the film layer is between 5 µm and 100 µm, with some particular embodiments being between 12.7 µm and 25.1 µm.

To electrically interconnect the control logic dies 206 and the transducers 212, in an embodiment, the flex circuit 214 further includes conductive traces 216 formed on the film layer that carry signals between the control logic dies 206 and the transducers 212. In particular, the conductive traces 216 providing communication between the control logic dies 206 and the transducers 212 extend along the flex circuit 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 112 when the conductors 218 of the cable 112 are mechanically and electrically coupled to the flex circuit 214. Suitable materials for the conductive traces 216 include copper, gold, aluminum, silver, tantalum, nickel, and tin, and may be deposited on the flex circuit 214 by processes such as sputtering, plating, and etching. In an embodiment, the flex circuit 214 includes a chromium adhesion layer. The width and thickness of the conductive traces 216 are selected to provide proper conductivity and resilience when the flex circuit 214 is rolled. In that regard, an exemplary range for the thickness of a conductive trace 216 and/or conductive pad is between 10-50 µm. For example, in an embodiment, 20 µm conductive traces 216 are separated by 20 µm of space. The width of a conductive trace 216 on the flex circuit 214 may be further determined by the width of the conductor 218 to be coupled to the trace/pad.

The flex circuit 214 can include a connection interface 220 in some embodiments. The connection interface 220 can be a location of the flex circuit 214 where the conductors 218 of the cable 112 are coupled to the flex circuit 214. For example, the bare conductors of the cable 112 are electrically coupled to the flex circuit 214 at the connection interface 220. The connection interface 220 can be a tab extending from the main body of flex circuit 214. In that regard, the main body of the flex circuit 214 can refer collectively to the transducer region 204, controller region 208, and the transition region 210. In the illustrated embodiment, the connection interface 220 extends from the proximal portion 222 of the flex circuit 214. In other embodiments, the connection interface 220 is positioned at other parts of the flex circuit 214, such as the distal portion 221, or the flex circuit 214 omits the connection interface 220. A value of a dimension of the tab or connection interface 220, such as a width 224, can be less than the value of a dimension of the main body of the flex circuit 214, such as a width 226. In some embodiments, the substrate forming the connection interface 220 is made of the same material(s) and/or is similarly flexible as the flex circuit 214. In other embodiments, the connection interface 220 is made of different materials and/or is comparatively more rigid than the flex circuit 214. For example, the connection interface 220 can be made of a plastic, thermoplastic, polymer, hard polymer, etc., including polyoxymethylene (e.g., DELRIN^{®}), polyether ether ketone (PEEK), nylon, and/or other suitable materials. As described in greater detail herein, the support member 230, the flex circuit 214, the connection interface 220 and/or the conductor(s) 218 can be variously configured to facilitate efficient manufacturing and operation of the imaging assembly 110.

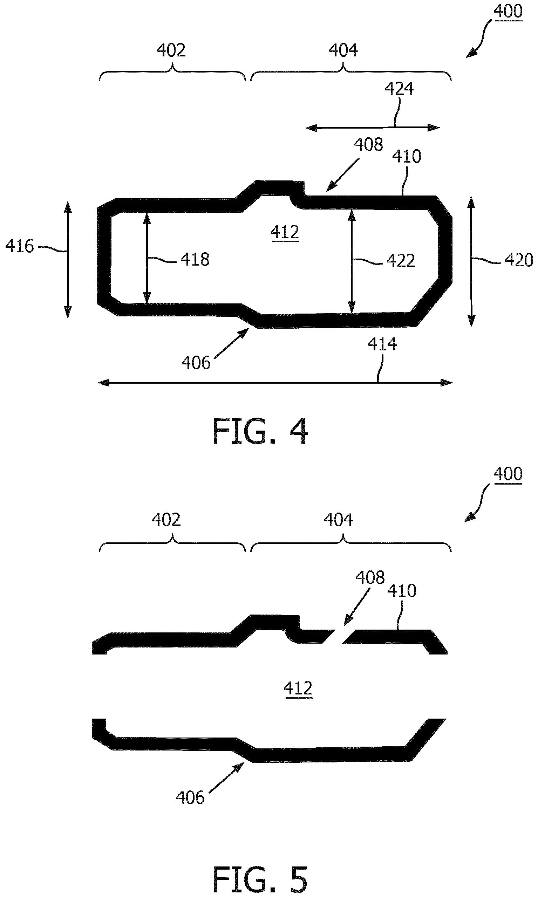

In some instances, the imaging assembly 110 is transitioned from a flat configuration (Fig. 2) to a rolled or more cylindrical configuration (Figs. 3 and 4). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND TRANSDUCER ASSEMBLY HAVING A FLEXIBLE SUBSTRATE".

As shown in Fig. 3, the flex circuit 214 is positioned around the support member 230 in the rolled configuration. Fig. 3 is a diagrammatic side view with the flex circuit 214 in the rolled configuration around the support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014 (published as the above-mentioned US 2015/0305710 A1). The support member 230 can have a distal section 232, a proximal section 234, and a lumen 236 extending longitudinally therethrough. The lumen 236 can be in communication with the exit port 116 and is sized and shaped to receive the guide wire 118 (Fig. 1). The support member 230 can be manufactured accordingly to any suitable process. For example, the support member 230 can be machined, such as by removing material from a blank to shape the support member 230, or molded, such as by an injection molding process. In some embodiments, the support member 230 may be integrally formed as a unitary structure, while in other embodiments the support member 230 may be formed of different components.

The support member 230 can be substantially cylindrical in some embodiments. Other shapes of the support member 230 are also contemplated including geometrical, non-geometrical, symmetrical, non-symmetrical, cross-sectional profiles. Different portions the support member 230 can be variously shaped in other embodiments. For example, the proximal portion of the support member 230 can have a larger outer diameter than the outer diameters of the distal portion or a central portion extending between the distal and proximal portions. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter. In some embodiments, the imaging assembly 110 in the rolled configuration can have a hexagonal outer geometry at least at the proximal portion of the imaging assembly 110 as shown and can be relatively stiff. In other embodiments, the imaging assembly 110 may have a geometrical cross-sectional profile having between 3 and 18 sides.

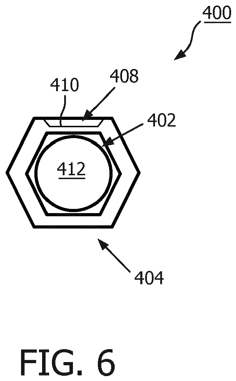

Figs. 4, 5, and 6 illustrate an exemplary embodiment of a support member 400 according to aspects of the present disclosure. The support member 400 can be used by the IVUS device 102. For example, the support member 400 can be placed over the junction or transition between the flexible elongate member 115 and the imaging assembly 110. Fig. 4 is a diagrammatic side view of the support member 400; Fig. 5 is a diagrammatic cross-sectional side view of the support member 400; and Fig. 6 is a diagrammatic end view of the support member 400. The support member 400 allows for a robust structural connection between the flexible elongate member and the ultrasound imaging assembly 110, while also facilitating a robust electrical connection between a communication cable 112 extending along a length of the intraluminal device to the ultrasound imaging assembly 110. Further, the support member 400 provides for a streamlined manufacturing process with consistently repeatable results. In contrast, the mechanical and electrical connections of the flexible elongate member and communication cable with the ultrasound imaging assembly of current intravascular ultrasound devices are often dependent on the skill of the person assembling the device and, therefore, can have high variability from device to device, reducing both the production yield during manufacturing and customer satisfaction due to variances between devices or device failure during use.

As shown, the support member 400 includes a proximal portion 402, a distal portion 404, and a transition 406 between the proximal portion 402 and the distal portion 404. The distal portion 404 includes an opening 408 extending through a wall of the support member 400 to an outer surface 410 of the support member 400 to a lumen 412 extending longitudinally through the support member 400. As will be discussed further below, the opening 408 can provide a passageway for a communication cable, such as a cable 112, to extend through the support member 400 for connection to the ultrasound imaging assembly 110. Further, the outer surface 410 of the support member 400 can provide a support surface or support structure for a connection portion of the ultrasound imaging assembly 110. In this regard, the connection portion of the ultrasound imaging assembly 110 may be a flex circuit in some implementations.

The proximal portion 402 of the support member 400 can be sized and shaped to mate with a distal section of the flexible elongate member, while the distal portion 404 of the support can be sized and shaped to mate with a proximal section of the ultrasound imaging assembly 110. Accordingly, in the illustrated embodiment the proximal portion 402 has a circular cross-sectional profile to match a corresponding circular cross-sectional profile of the flexible elongate member, while the distal portion 404 has a hexagonal cross-sectional profile to match a corresponding hexagonal profile of a proximal section of the ultrasound imaging assembly 110. As shown, the proximal portion 402 can have a different cross-sectional profile than the distal portion 404. In this regard, in some implementations the proximal portion 402 has a circular cross-sectional profile, while the distal portion 404 has a geometrical cross-sectional profile having between 3 and 18 sides (e.g., triangle, rectangle, square, pentagon, hexagon, octagon, decagon, etc.), including between 4 and 12 sides. However, it is understood that each of the proximal portion 402 and the distal portion 404 may have any suitable cross-sectional profile configured to mate with the flexible elongate member and ultrasound imaging assembly 110, respectively. By matching the geometry of the proximal portion 402 to the geometry of the flexible elongate member (e.g., the outer shaft) and the geometry of the distal portion 404 to the geometry of the imaging assembly, the support member 400 can bridge the distinct difference between the diameters and shapes of the flexible elongate member and the imaging assembly.

Generally, the size of the support member 400 is determined based on a desired outer diameter of the intravascular device and the associated sizes and structures of the flexible elongate member and the ultrasound imaging assembly 110. Accordingly, in some implementations the support member 400 has an overall length 414 between about 5 mm and about 15 mm, including between about 7 mm and about 10 mm. In this regard, the proximal portion 402 can have a length between about 1 mm and about 5 mm, the distal portion 404 can have a length between about 0.5 mm and about 5 mm, and the transition 406 can have a length between about 0 mm and about 1 mm. The proximal portion 402 can have an outer diameter or height 416 that matches a desired outer diameter of the intraluminal device. Accordingly, in some implementations the outer diameter of height 416 is between about 0.014" (0.35 mm) and about 0.042" (1.07 mm), with some particular embodiments between about 0.036" (0.91 mm) and about 0.039" (0.99 mm). The lumen 412 within the proximal portion 402 can have a diameter or height 418 between about 0.010" (0.25 mm) and about 0.035" (0.89 mm), with some particular embodiments between about 0.030" (0.76 mm) and about 0.033" (0.84 mm). The distal portion 404 can have an outer diameter or height 420 that matches a desired outer diameter of the intraluminal device. Accordingly, in some embodiments the outer diameter or height 420 is between about 0.014" (0.35 mm) and about 0.042" (1.07 mm), with some particular embodiments between about 0.034" (0.86 mm) and about 0.035" (0.89 mm). The lumen 412 within the distal portion 404 can have a diameter or height 422 between about 0.010" (0.25 mm) and about 0.035" (0.89 mm), with some particular embodiments between about 0.030" (0.76 mm) and about 0.0305" (0.77 mm). The opening 408 can be sized and shaped to facilitate passage of a communication cable therethrough. Accordingly, in some instances the opening 408 has a diameter or width between about 0.010" (0.25 mm) and about 0.030" (0.76 mm). The outer surface 410 can have a length 424 between about 0.25 mm and about 2 mm, with some particular embodiments between about 0.5 mm and about 1 mm. The support member 400 can be formed of any suitable material, including without limitation polyether block amides (such as Pebax), high density polyethylene, low density polyethylene, and polyimide. In some implementations the support member 400 is formed of a material having a flexibility or durometer between that of a distal end section of the flexible elongate member and that of a proximal end section of the imaging assembly such that the support member 400 provides a transition in stiffness between the distal end section of the flexible elongate member and the proximal end section of the imaging assembly. In some implementations, the support member 400 is formed as a molded piece.

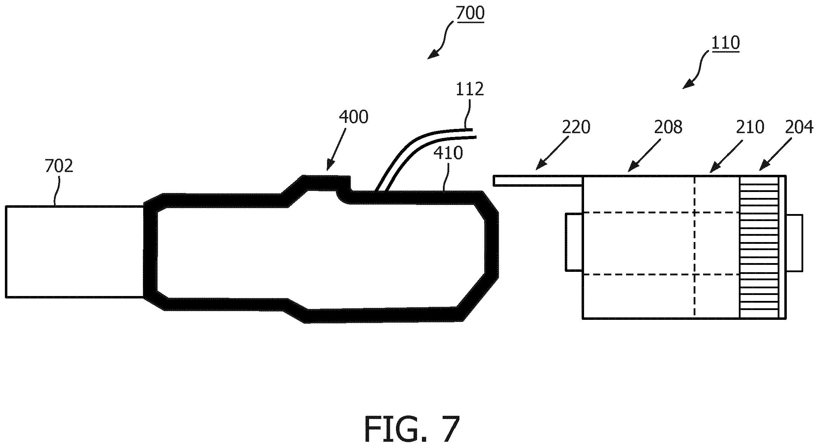

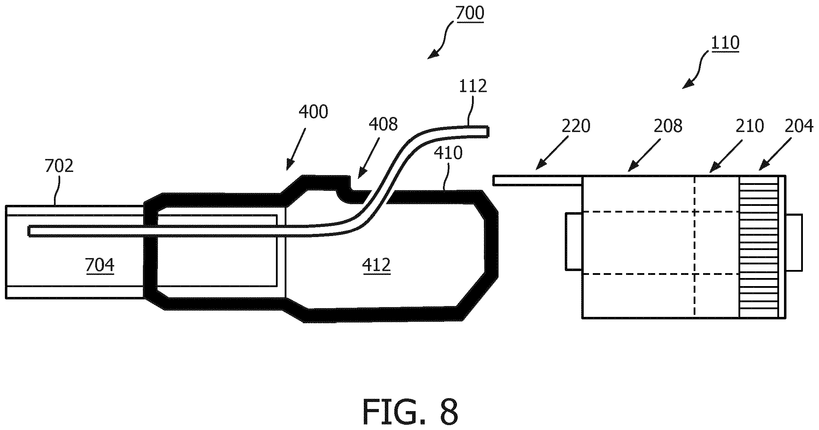

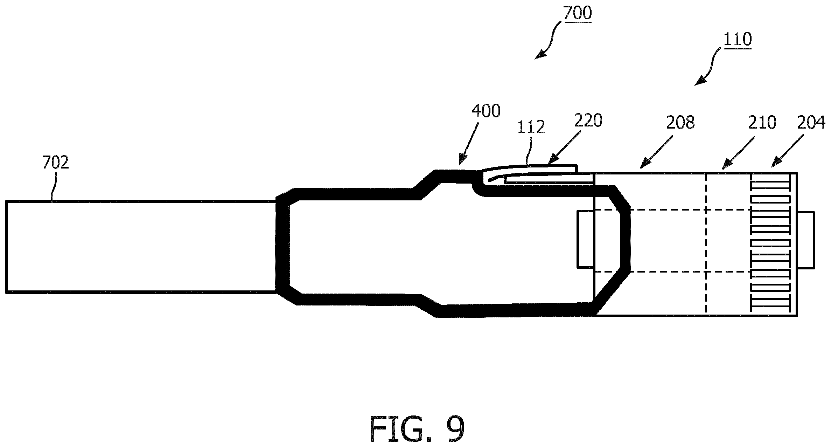

Referring now to Figs. 7, 8, and 9, shown therein are aspects of assembling a distal portion 700 of an intraluminal imaging device according to the present disclosure. In particular, Fig. 7 is a diagrammatic side view of the distal portion 700 of the intraluminal imaging device in an assembly stage; Fig. 8 is a diagrammatic partial cross-sectional side view of the distal portion 700 of the intraluminal imaging device in the assembly stage; and Fig. 9 is a diagrammatic side view of the assembled distal portion 700 of the intraluminal imaging device. As shown in Figs. 7 and 8, the support member 400 can be positioned over a distal section 702 of a flexible elongate member such as the flexible elongate member 115. In this regard, the distal section 702 of the flexible elongate member can include a lumen 704 extending along all or a portion of its length. A communication cable 112 may extend through the lumen 704. The communication cable 112 may extend at least partially through the lumen 412 of the support member 400 and out through the opening 408 of the support member 400, as best seen in Fig. 8.

As shown in Fig. 9, the imaging assembly 110 can be coupled with the support member 400 and distal section 702 of the flexible elongate member. In some implementations, the support member 400 is fixedly attached to the distal section 702 of the flexible elongate member (e.g., via adhesive, epoxy, melting, curing, mechanical connection, press-fit, etc.) prior to the imaging assembly 110 being coupled to the support member 400. In some implementations, the support member 400 can be assembled by positioning or sliding the support member 400 over the distal section 702 of the flexible elongate member. The distal section 702 of the flexible elongate member can be coupled or bonded to the imaging assembly 110. After coupling the imaging assembly 110 to the distal section 702 of the flexible elongate member, the support member 400 can be positioned over at least a proximal portion of the imaging assembly 110. Thus, the support member 400 at least partially overlaps with the distal section 702 of the flexible elongate member and the proximal section of the imaging assembly. For example, the support member 400 can be coupled to the imaging assembly 110 such that connection interface 220 mates with the outer surface 410 of the support member 400. In this regard, the outer surface 410 can provide mechanical support to the connection interface 220, which may be a portion of a flex circuit. The conductors of the communication cable 112 may be electrical coupled (e.g., soldering, welding, etc.) to conductive pads or traces of the connection interface 220 either prior to or after coupling the imaging assembly to the support member 400. In some instances, an epoxy, adhesive, or other sealant is applied over the electrical connections between the conductors of the communication cable 112 and the conductive pads or traces of the connection interface 220 to seal, secure, and/or protect the electrical connections.

The imaging assembly 110 can be fixedly attached to the support member 400 and distal section 702 of the flexible elongate member by an adhesive or epoxy. For example, in some instances an adhesive or epoxy is introduced into the lumen 412 of the support member 400 and/or the lumen 704 of the flexible elongate member such that the adhesive or epoxy bonds a proximal section of the imaging assembly to the support member 400 and flexible elongate member. By having the adhesive or epoxy within the defined lumen(s) of the support member 400 and flexible elongate member a repeatable and consistent manufacturing process can be implemented using suitable volumes of adhesive/epoxy, curing temperatures, and/or curing times to provide a robust mechanical connection. Further, because the adhesive/epoxy is positioned within the lumen(s) of the support member 400 and flexible elongate member, the outer profile of the intraluminal imaging device can be more consistent from device to device.

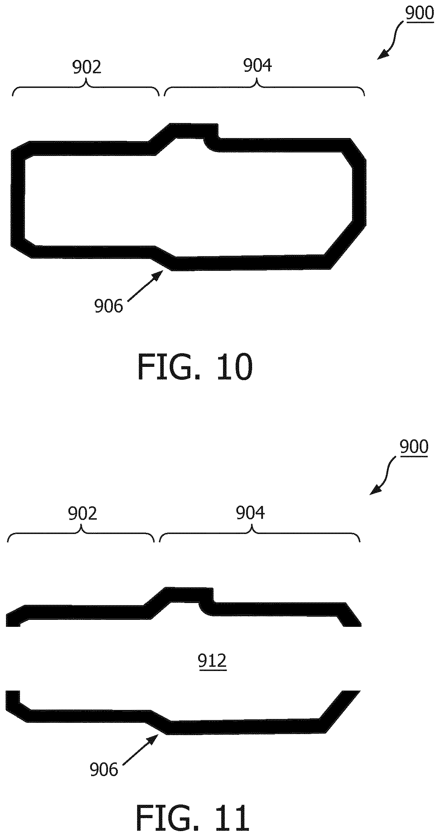

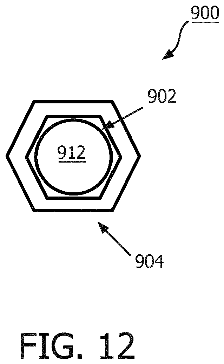

Figs. 10, 11, and 12 illustrate an exemplary embodiment of a support member 900 according to aspects of the present disclosure. Fig. 10 is a diagrammatic side view of the support member 900; Fig. 11 is a diagrammatic cross-sectional side view of the support member 900; and Fig. 12 is a diagrammatic end view of the support member 900. The support member 900 is similar in many respects to support member 400 described above. As shown, the support member 900 includes a proximal portion 902, distal portion 904, a transition 906 between the proximal and distal portions, and a lumen 912. The primary difference between support member 900 and support member 400 is that the support member 900 does not include an opening or associated outer surface for mounting the connection interface 220 of the imaging assembly 110. Instead, the support member 900 is configured to receive the proximal section of the imaging assembly 110 entirely within the lumen 912. As a result, the electrical connections between the communication cable 112 and the imaging assembly 110 can be protected by the support member 900.

In this regard, the imaging assembly 110 can be coupled and/or fixedly attached to the support member 900 and distal section 702 of the flexible elongate member in a similar manner as described above for support member 400. For example, in some instances an adhesive or epoxy is introduced into the lumen 912 of the support member 900 and/or the lumen 704 of the flexible elongate member such that the adhesive or epoxy bonds a proximal section of the imaging assembly 110 to the support member 900 and flexible elongate member. By having the adhesive or epoxy within the defined lumen(s) of the support member 900 and flexible elongate member a repeatable and consistent manufacturing process can be implemented using suitable volumes of adhesive/epoxy, curing temperatures, and/or curing times to provide a robust mechanical connection. Further, because the adhesive/epoxy is positioned within the lumen(s) of the support member 400 and flexible elongate member, the outer profile of the intraluminal imaging device can be more consistent from device to device. As a result, the support member 900 provides a robust structural connection between the flexible elongate member and the ultrasound imaging assembly 110, while facilitating a secure and strong electrical connection between the communication cable 112 and the ultrasound imaging assembly 110.

The disclosed embodiments can provided several benefits. For example, the employments of the support members 400 and 900 can provide a robust joint with a low profile and a smooth transition at the junction between the imaging assembly 110 and the flexible elongate member 115. The support members 400 and 900 can provide support to the conductive traces 216 at the junction and prevent the conductive traces 216 from excessive flexing when in use. Thus, the disclose embodiments can improve durability, strength, and navigational capability of the IVUS device 102. In addition, the matching of the geometry of the support members 400 and 900 to the geometries of the flexible elongate member 115 and the imaging assembly can provide electrical seal and protection to the electrical connections at the transition region. As such, the disclosed embodiments can improve imaging quality and reliability. Further, the employments of the support members 400 and 900 can mitigate variability from the manual soldering and assembly process.

## Claims

1. An intraluminal imaging device (102), comprising:
a flexible elongate member (115) configured for positioning within a vessel (120) of a patient;
a support member (400) coupled to the flexible elongate member (115); and
an ultrasound imaging assembly (110) coupled to the support member (400), comprising a transducer array (124) formed in a transducer region (204) and transducer control logic dies (206) formed in a control region (208) with a transition region (210) disposed therebetween, wherein the transducer array and the transducer control logic dies are mounted on a flex circuit (214) positioned around a second support member in a rolled configuration;
wherein the support member (400) includes a proximal section (402) with a first cross-sectional profile sized and shaped to mate with a distal portion of the flexible elongate member (115) and a distal section (404) with a second cross-sectional profile sized and shaped to mate with the proximal section of the ultrasound imaging assembly (110), wherein the first cross-sectional profile is circular and the second cross-sectional profile is a geometrical shape including between four and twelve sides; and
wherein the support member bridges the distinct difference between the diameters and shapes of the flexible elongate member and the ultrasound imaging assembly.

2. The intraluminal imaging device (102) of claim 1, wherein the support member (400) further includes a lumen (412) and an opening (408) extending through a wall in communication with the lumen (412), the opening (408) configured to receive a communication cable (112).

3. The intraluminal imaging device (102) of claim 2, wherein a connection interface (220) of the imaging assembly (110) is mounted to an outer surface (410) of the support member (400) adj acent to the opening (408).

4. The intraluminal imaging device (102) of claim 3, wherein the connection interface (220) includes a flex circuit (214).

5. The intraluminal imaging device (102) of claim 3, further comprising the communication cable (112) extending through the opening (408) and electrically coupled to the connection interface (220) of the ultrasound imaging assembly (110).

6. The intraluminal imaging device (102) of claim 1, wherein the support member (400) includes a lumen (412) and wherein an adhesive within the lumen (412) fixedly secures the ultrasound imaging assembly (110) to the support member (400).

7. The intraluminal imaging device (102) of claim 6, wherein a connection interface (220) of the ultrasound imaging assembly (110) is positioned within the lumen (412).

8. A method of forming an intraluminal imaging device (102), comprising:
providing a support member (400) comprising a proximal section with a first cross-sectional profile sized and shaped to mate with a distal portion of a flexible elongate member and a distal section with a second cross-sectional profile sized and shaped to mate with a proximal section of an ultrasound imaging assembly, wherein the first cross-sectional profile is circular and the second cross-sectional profile is a geometrical shape including between four and twelve sides;
coupling the distal portion of the flexible elongate member (115) configured for positioning within a vessel (120) of a patient to the proximal section of the support member (400); and
coupling the proximal section of the ultrasound imaging assembly (110) to the distal section of the support member (400); wherein the ultrasound imaging assembly comprises a transducer array (124) formed in a transducer region (204) and transducer control logic dies (206) formed in a control region (208) with transition region (210) disposed therebetween, wherein the transducer array and transducer control logic dies are mounted on a flex circuit (214) positioned around a second support member in a rolled configuration; and
wherein the support member bridges the distinct difference between the diameters and shapes of the flexible elongate member and the ultrasound imaging assembly.

9. The method of claim 8, wherein the support member (400) further includes a lumen (412) and an opening (408) extending through a wall in communication with the lumen (412), the method further comprising introducing a communication cable (112) through the opening (408).

10. The method of claim 9, further comprising mounting a connection interface (220) of the ultrasound imaging assembly (110) to an outer surface of the support member (400) adjacent to the opening.

## Patentansprüche

1. Intraluminale Bildgebungsvorrichtung (102), umfassend:
ein flexibles längliches Element (115), das zur Positionierung in einem Gefäß (120) eines Patienten konfiguriert ist;
ein Trägerelement (400), das mit dem flexiblen länglichen Element (115) verbunden ist; und
eine Ultraschallbildgebungsanordnung (110), die mit dem Trägerelement (400) gekoppelt ist und ein Wandlerarray (124) umfasst, das in einem Wandlerbereich (204) ausgebildet ist, und Wandlersteuerlogikchips (206), die in einem Steuerbereich (208) mit einem dazwischen angeordneten Übergangsbereich (210) ausgebildet sind, wobei das Wandlerarray und die Wandlersteuerlogikchips auf einer flexiblen Schaltung (214) montiert sind, die in einer gerollten Konfiguration um ein zweites Trägerelement herum positioniert ist;
wobei das Trägerelement (400) einen proximalen Abschnitt (402) mit einem ersten Querschnittsprofil umfasst, das so bemessen und geformt ist, dass es mit einem distalen Abschnitt des flexiblen länglichen Elements (115) zusammenpasst, und einen distalen Abschnitt (404) mit einem zweiten Querschnittsprofil umfasst, das so bemessen und geformt ist, dass es mit dem proximalen Abschnitt der Ultraschallbildgebungsanordnung (110) zusammenpasst, wobei das erste Querschnittsprofil kreisförmig ist und das zweite Querschnittsprofil eine geometrische Form mit vier bis zwölf Seiten ist; und
wobei das Trägerelement den deutlichen Unterschied zwischen den Durchmessern und Formen des flexiblen länglichen Elements und der Ultraschallbildgebungsanordnung überbrückt.

2. Intraluminale Bildgebungsvorrichtung (102) nach Anspruch 1, wobei das Trägerelement (400) außerdem ein Lumen (412) und eine Öffnung (408) umfasst, die sich durch eine Wand in Verbindung mit dem Lumen (412) erstreckt, wobei die Öffnung (408) konfiguriert ist, um ein Kommunikationskabel (112) aufzunehmen.

3. Intraluminale Bildgebungsvorrichtung (102) nach Anspruch 2, wobei eine Verbindungsschnittstelle (220) der Bildgebungsanordnung (110) an einer Außenfläche (410) des Trägerelements (400) angrenzend an die Öffnung (408) montiert ist.

4. Intraluminale Bildgebungsvorrichtung (102) nach Anspruch 3, wobei die Verbindungsschnittstelle (220) eine flexible Schaltung (214) umfasst.

5. Intraluminale Bildgebungsvorrichtung (102) nach Anspruch 3, ferner umfassend das Kommunikationskabel (112), das sich durch die Öffnung (408) erstreckt und elektrisch mit der Verbindungsschnittstelle (220) der Ultraschallbildgebungsanordnung (110) gekoppelt ist.

6. Intraluminale Bildgebungsvorrichtung (102) nach Anspruch 1, wobei das Trägerelement (400) ein Lumen (412) umfasst und wobei ein Klebstoff innerhalb des Lumens (412) die Ultraschallbildgebungsanordnung (110) fest am Trägerelement (400) befestigt.

7. Intraluminale Bildgebungsvorrichtung (102) nach Anspruch 6, wobei eine Verbindungsschnittstelle (220) der Ultraschallbildgebungsanordnung (110) innerhalb des Lumens (412) positioniert ist.

8. Verfahren zum Bilden einer intraluminalen Bildgebungsvorrichtung (102), umfassend:
Bereitstellen eines Trägerelements (400), umfassend einen proximalen Abschnitt mit einem ersten Querschnittsprofil, das so bemessen und geformt ist, dass es mit einem distalen Abschnitt eines flexiblen länglichen Elements zusammenpasst, und einen distalen Abschnitt mit einem zweiten Querschnittsprofil, das so bemessen und geformt ist, dass es mit einem proximalen Abschnitt einer Ultraschallbildgebungsanordnung zusammenpasst, wobei das erste Querschnittsprofil kreisförmig ist und das zweite Querschnittsprofil eine geometrische Form mit vier bis zwölf Seiten ist;
Koppeln des distalen Abschnitts des flexiblen länglichen Elements (115), das zur Positionierung innerhalb eines Gefäßes (120) eines Patienten konfiguriert ist, mit dem proximalen Abschnitt des Trägerelements (400); und
Koppeln des proximalen Abschnitts der Ultraschallbildgebungsanordnung (110) mit dem distalen Abschnitt des Trägerelements (400); wobei die Ultraschallbildgebungsanordnung ein Wandlerarray (124), das in einem Wandlerbereich (204) ausgebildet ist, und Wandlersteuerlogikchips (206) umfasst, die in einem Steuerbereich (208) mit dazwischen angeordnetem Übergangsbereich (210) ausgebildet sind, wobei das Wandlerarray und die Wandlersteuerlogikchips auf einer flexiblen Schaltung (214) montiert sind, die in einer gerollten Konfiguration um ein zweites Trägerelement herum positioniert ist, und
wobei das Trägerelement den deutlichen Unterschied zwischen den Durchmessern und Formen des flexiblen länglichen Elements und der Ultraschallbildgebungsanordnung überbrückt.

9. Verfahren nach Anspruch 8, wobei das Trägerelement (400) außerdem ein Lumen (412) und eine Öffnung (408) umfasst, die sich durch eine Wand erstreckt und mit dem Lumen (412) in Verbindung steht, wobei das Verfahren weiterhin das Einführen eines Kommunikationskabels (112) durch die Öffnung (408) umfasst.

10. Verfahren nach Anspruch 9, das ferner das Anbringen einer Verbindungsschnittstelle (220) der Ultraschallbildgebungsanordnung (110) an einer Außenfläche des Trägerelements (400) neben der Öffnung umfasst.

## Revendications

1. Dispositif d'imagerie intraluminale (102), comprenant :
un élément allongé flexible (115) configuré pour être positionné à l'intérieur d'un vaisseau (120) d'un patient;
un élément de support (400) couplé à l'élément allongé flexible (115); et
un ensemble d'imagerie ultrasonore (110) couplé à l'élément de support (400), comprenant un réseau de transducteurs (124) formé dans une région de transducteurs (204) et des puces logiques de commande de transducteurs (206) formées dans une région de commande (208) avec un une région de transition (210) disposée entre celles-ci, où le réseau de transducteurs et les puces logiques de commande des transducteurs sont montés sur un circuit flexible (214) positionné autour d'un second élément de support dans une configuration roulée;
où l'élément de support (400) comprend une section proximale (402) avec un premier profil en coupe transversale dimensionné et façonné pour s'accoupler avec une partie distale de l'élément allongé flexible (115) et une section distale (404) avec un second profil en coupe transversale dimensionné et façonné pour s'accoupler avec la section proximale de l'ensemble d'imagerie ultrasonore (110), où le premier profil en coupe transversale est circulaire et le second profil en coupe transversale est une forme géométrique comprenant entre quatre et douze côtés; et
où l'élément de support comble la différence nette entre les diamètres et les formes de l'élément allongé flexible et de l'ensemble d'imagerie ultrasonore.

2. Dispositif d'imagerie intraluminale (102) selon la revendication 1, dans lequel l'élément de support (400) comprend en outre un lumen (412) et une ouverture (408) s'étendant à travers une paroi en communication avec le lumen (412), l'ouverture (408) étant configuré pour recevoir un câble de communication (112).

3. Dispositif d'imagerie intraluminale (102) selon la revendication 2, dans lequel une interface de connexion (220) de l'ensemble d'imagerie (110) est montée sur une surface externe (410) de l'élément de support (400) adjacente à l'ouverture (408).

4. Dispositif d'imagerie intraluminale (102) selon la revendication 3, dans lequel l'interface de connexion (220) comprend un circuit flexible (214).

5. Dispositif d'imagerie intraluminale (102) selon la revendication 3, comprenant en outre le câble de communication (112) s'étendant à travers l'ouverture (408) et couplé électriquement à l'interface de connexion (220) de l'ensemble d'imagerie ultrasonore (110) .

6. Dispositif d'imagerie intraluminale (102) selon la revendication 1, dans lequel l'élément de support (400) comprend un lumen (412) et dans lequel un adhésif à l'intérieur du lumen (412) fixe solidement l'ensemble d'imagerie ultrasonore (110) à l'élément de support (400) .

7. Dispositif d'imagerie intraluminale (102) selon la revendication 6, dans lequel une interface de connexion (220) de l'ensemble d'imagerie ultrasonore (110) est positionnée à l'intérieur du lumen (412).

8. Procédé de formation d'un dispositif d'imagerie intraluminale (102), consistant à:
fournir un élément de support (400) comprenant une section proximale avec un premier profil en coupe transversale dimensionné et façonné pour s'accoupler avec une partie distale d'un élément allongé flexible et une section distale avec un second profil en coupe transversale dimensionné et façonné pour s'accoupler avec une section proximale d'un ensemble d'imagerie ultrasonore, où le premier profil en coupe transversale est circulaire et le second profil en coupe transversale est une forme géométrique comprenant entre quatre et douze côtés;
coupler la partie distale de l'élément allongé flexible (115) configuré pour être positionné à l'intérieur d'un vaisseau (120) d'un patient à la section proximale de l'élément de support (400); et
coupler la section proximale de l'ensemble d'imagerie ultrasonore (110) à la section distale de l'élément de support (400); où l'ensemble d'imagerie ultrasonore comprend un réseau de transducteurs (124) formé dans une région de transducteurs (204) et des puces logiques de commande de transducteurs (206) formées dans une région de commande (208) avec une région de transition (210) disposée entre celles-ci, où le réseau de transducteurs et les puces logiques de commande des transducteurs sont montées sur un circuit flexible (214) positionné autour d'un second élément de support dans une configuration roulée; et
où l'élément de support comble la différence nette entre les diamètres et les formes de l'élément allongé flexible et de l'ensemble d'imagerie ultrasonore.

9. Procédé selon la revendication 8, dans lequel l'élément de support (400) comprend en outre un lumen (412) et une ouverture (408) s'étendant à travers une paroi en communication avec le lumen (412), le procédé comprenant en outre l'introduction d'un câble de communication (112) à travers l'ouverture (408).

10. Procédé selon la revendication 9, comprenant en outre le montage d'une interface de connexion (220) de l'ensemble d'imagerie ultrasonore (110) sur une surface externe de l'élément de support (400) adjacente à l'ouverture.
